# EUROPEAN PATENT APPLICATION

(11) **EP 2 226 353 A1**
(43) Date of publication of application: **08.09.2010**
(21) Application number: 08865747.3
(22) Date of filing: 25.12.2008
(51) Int. Cl.: C08J 11/14, C08G 63/88, C08J 11/08, C08J 11/16

(54) **METHOD OF DECOMPOSING THERMOSET RESIN AND RECOVERING PRODUCT OF DECOMPOSITION**

(30) Priority: 25.12.2007 JP 2007332514
(71) Applicant: Panasonic Electric Works Co., Ltd, Kadoma-shi Osaka 571-8686 (JP); International Center for Environmental Technology Transfer, Yokkaichi-shi, Mie 5121211 (JP)
(72) Inventor: SHIBATA, Keishi, Kadoma-shi Osaka 571-8686 (JP); NAKAGAWA, Takaharu, Kadoma-shi Osaka 571-8686 (JP); YOSHIMURA, Takeshi, Kadoma-shi Osaka 571-8686 (JP)
(74) Representative: von Kreisler Selting Werner
(86) International application number: PCT/JP2008/073557
(87) International publication number: WO 2009/081974

(57) **Abstract**

The present invention relates to a method for decomposing a thermosetting resin to provide decomposed products, which can be easily separated, efficiently collected and recycled. Specifically, the present invention relates to a method for decomposing a thermosetting resin comprising a polyester moiety and a crosslinking moiety therewith to provide decomposed products to be collected and recycled, which comprises steps of:
(A) decomposing the thermosetting resin by using of subcritical water in the presence of an alkali;
(B) subjecting the decomposed products to a solid-liquid separation to provide an aqueous solution comprising a salt of a compound with an alkali, wherein the compound comprises an acid residue derived from the polyester moiety and a residue derived from the crosslinking moiety; and
(C) adding an acid to the aqueous solution to precipitate the compound, and adding thereto a solvent, which can dissolve the compound and which is water insoluble, to dissolve the precipitated compound into the solvent to provide a solution comprising the compound to be collected.

## Description

### Technical Field

The present application is filed with claiming the priority of the Japanese Patent Application No. 2007-332514, which was filed on December 25, 2007, the entire contents of which are incorporated herein by the reference.
The present invention relates to a method for decomposing a thermosetting resin by using of subcritical water to provide decomposed products to be collected and recycled, such as monomers, styrene-fumaric acid copolymers, etc.

### Background Art

Most of waste plastics hitherto have been dumped by reclaiming lands with the same or incinerating the same, and have never been used as a useful resource. This waste disposal by way of reclaiming the lands has difficulties in the ensuring of the sites to be reclaimed, and in stable hardening of such sites. On the other hand, the disposal by way of incinerating the waste plastics has disadvantages, such as the damage to the incinerators, generation of organic gases and offensive odors, and emission of CO₂.

To solve these problems, the Containers and Packaging Recycling Law was instituted in 1995 in Japan, so as to obligate the recovering and recycling of plastics. This trend of recovering and recycling products containing plastics is prevailing in association with the enforcement of a variety of recycling laws.

Recently, under such circumstances, there is a trial such waste plastics are to be recycled and reused as material resources. For example, the following patent literatures 1-5 suggest methods for decomposing the plastics by using of supercritical or subcritical water, as a reaction media, in order to provide decomposed products to be collected and then recycled.

Since these methods, however, randomly decompose the plastics, it is difficult to provide a curtain quality of decomposed products.

In order to solve such problems, for example, the following patent literature 6 suggests a method for decomposing, with subcritical water, a thermosetting resin of a polyester composed of a polyalcohol and a polybasic acid, which has been crosslinked by a crosslinking agent, under the thermal decomposition temperature of the thermosetting resin to provide monomers to be recycled as materials for newly producing a thermosetting resin as well as styrene-fumaric acid copolymers.
**Patent Literature 1:** JP-T-56-501205
**Patent Literature 2:** JP-A-57-4225
**Patent Literature 3:** JP-A-5-31000
**Patent Literature 4:** JP-A-6-279762
**Patent Literature 5:** JP-A-10-67991
**Patent Literature 6:** WO 2005/092962

### Disclosure of Intention

### Problems to be solved by the invention

The patent literature 6 discloses a method for decomposing a thermosetting resin with an alkali-containing subcritical water to provide styrene-fumaric acid copolymers. The styrene-fumaric acid copolymers resulted from this decomposition reaction are dissolved, as salts, in an aqueous solution. Subsequently, the styrene-fumaric acid copolymers are precipitated by adding an acid, such as hydrochloric acid and sulfuric acid, to the aqueous solution. The precipitates are separated off, and then collected.

Herein, during the separation and collection of the styrene-fumaric acid copolymers on a filter of an apparatus such as a filter press, there is a problem associated with the filtration such that clogging occurs on the filter, which prevents the continuous filtration. In case of a solid-liquid separation for the styrene-fumaric acid copolymers in a solid-liquid separator such as a centrifugal separator, there are problems such that some styrene-fumaric acid copolymers are remains in the aqueous phase, and that the styrene-fumaric acid copolymers are coagulated to provide the separator with clogging therewith. In any solid-liquid separations, the styrene-fumaric acid copolymers after the separation contain any moisture. Therefore, it takes much time to remove the moisture by drying, which is also problem to be solved.

Accordingly, objects of the present invention, in order to solve the above-described problems in the art, consists in a provision of a method for decomposing a thermosetting resin to provide decomposed products which contain compounds comprising an acid residue derived from the polyester moiety of the resin and a residue derived from the crosslinking moiety of the resin (e.g., styrene-fumaric acid copolymers), which can be easily separated and effectively collected, and then recycled.

### Means for solving problems

The present invention in order to solve the above-described problems comprises:
[1] a method for decomposing a thermosetting resin comprising a polyester moiety and a crosslinking moiety therewith to provide decomposed products to be collected and recycled, which comprises steps of:
   (A) decomposing the thermosetting resin by using of subcritical water in the presence of an alkali;
   (B) subjecting the decomposed products to a solid-liquid separation to provide an aqueous solution comprising a salt of a compound with an alkali, wherein the compound comprises an acid residue derived from the polyester moiety and a residue derived from the crosslinking moiety; and
   (C) adding an acid to the aqueous solution to precipitate the compound, and adding thereto a solvent, which can dissolve the compound and which is water insoluble, to dissolve the precipitated compound into the solvent to provide a solution comprising the compound to be collected;
[2] the method according to the above item [1], wherein the solvent, which can dissolve the compound and which is water insoluble, comprises an alcohol
[3] the method according to the above item [1] or [2], wherein the step (c) further comprises adding a co-solvent, which can dissolve the compound and which is water soluble, together with the solvent, which can dissolve the compound and which is water insoluble.

### Effects of the invention

The present invention employs a solvent which is water insoluble and which can dissolve a compound comprising an acid residue derived from the polyester moiety of the thermosetting resin and a residue derived from the crosslinking moiety of the thermosetting resin (e.g., styrene-fumaric acid copolymer). Although the compound is precipitated in the aqueous solution, the solvent can dissolve the compound. The solvent phase containing the compound can be easily separated from the aqueous phase. Separation of the solvent phase from the aqueous phase can provide the compound dissolved in the solvent. Subsequently, the compound can be effectively collected.

In the method according to the present invention, the solvent, which can dissolve the compound and which is water insoluble, includes alcohols, which can effectively modify (or esterify) the compound in order to facilitate the modified process to produce a shrinkage inhibitor for a thermosetting resin.

In the method according to the present invention, together with the solvent which can dissolve the compound and which is water insoluble, a co-solvent, which can dissolve the compound and which is water soluble, can be added, which can accelerate the compound is dissolved into the solvent. Accordingly, the compound can be much further effectively yielded.

### Brief Description of Drawings

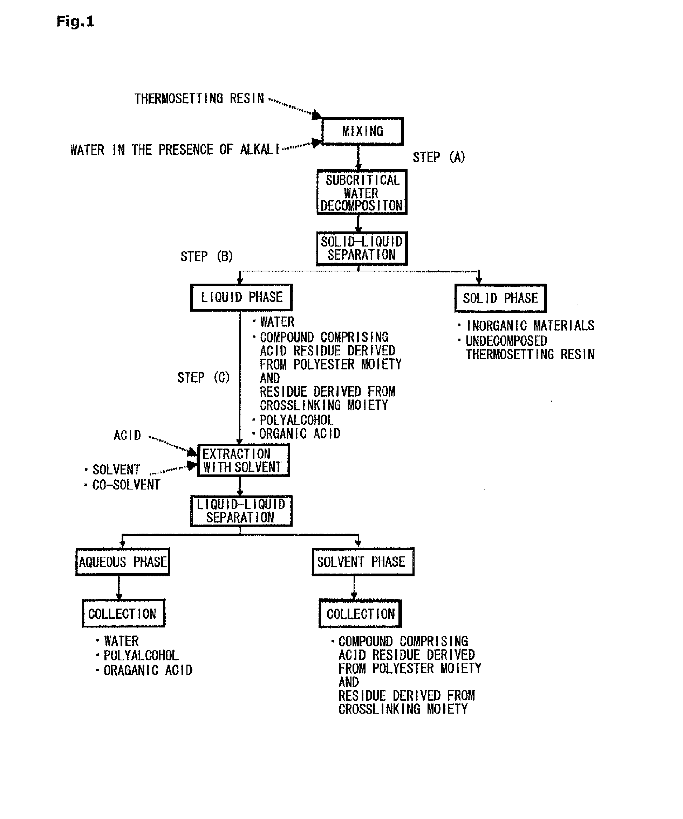

**Fig.1** is a flowchart showing one embodiment of the method according to the present invention comprising procedures and steps in order.

### Best Mode for Carrying Out the Invention

The present invention is described hereinafter in detail. The subjective thermosetting resin to be decomposed according to the present invention includes crosslinked polyester resins, which are obtainable by crosslinking of any polyesters, and which comprises a polyester moiety and a crosslinking moiety therewith.

The polyester moiety is derived from a polyester prepared by a polycondensation of a polyalcohol and a polybasic acid. Therefore, the polyester comprises a polyalcohol residues and a polybasic acid residue, both of which are attached together via an ester bond. The polyester moiety can comprise a double bond(s) which is/are contained in the starting unsaturated polybasic acid.

The crosslinking moiety bridges the above-described polyester moieties. For example, the crosslinking moiety can be derived from any crosslinking agent, but which is not particularly limited. The crosslinking moiety can be derived from a single crosslinking agent or a plurality of crosslinking agents which are polymerized to form an oligomer or a polymer. It is not particularly limited to wherein the crosslinking moiety and the polyester moiety are bonded together or how they are attached to each other.

Therefore, the wording of the "thermosetting resin comprising a polyester moiety and a crosslinking moiety therewith" means a thermosetting resin comprising a polyester essentially composed of a polyalcohol and a polybasic acid, which is crosslinked, or networked, via a crosslinking moiety (i.e., networked polyester resin). The thermosetting resin includes any resins exerting the above-described effects, which can be applied to the present invention. Therefore, there are no limitations on type of the resin, structure of the resin, type of the crosslinking moiety (or crosslinking agent), amount thereof, and degree of crosslinking, etc.

The thermosetting resins which can be applied to the present invention have been predominantly cured (or crosslinked) by means of heating, etc. The thermosetting resins may be uncured or partially cured resins which can be sufficiently cured (or crosslinked) by means of further heating or the like, and which can be applied to the present invention in order to exert the above-described effects.

The preferable thermosetting resin to be applied to the present invention includes an unsaturated polyester derived from a polyalcohol and an unsaturated polybasic acid, which has been crosslinked by means of a crosslinking agent to be formed into a networked polyester resin.

The starting polyalcohols to form the polyester moiety include, for example, glycols such as ethylene glycol, propylene glycol, neopentyl glycol, diethylene glycol and dipropylene glycol, etc. A single polyalcohol can be used alone. Alternatively, two or more polyalcohols can be used in any combination.

The starting polybasic acids to form the polyester moiety include, for example, aliphatic unsaturated dibasic acids such as maleic anhydride, maleic acid, fumaric acid, etc. A single polybasic acid can be used alone. Alternatively, two or more polybasic acids can be used in any combination. A saturated polybasic acid such as phthalic anhydride can be used in any combination with the unsaturated polybasic acid(s).

The crosslinking agent can crosslink any polyester which is a copolymer of the polyalcohol and the polybasic acid. The crosslinking agent includes styrene, etc. The other crosslinking agent such as polymerizable vinyl monomers (e.g., methyl methacrylate) can be used in a combination with the above-described crosslinking agent.

Herein, the subjective thermosetting resin to be decomposed according to the present invention can comprise another component(s) such as an inorganic filler (e.g., calcium carbonate, aluminum hydroxide), and a glass fiber (e.g., chopped strand which is produced by cutting a roving).

The present invention comprises the following steps (A)-(C), wherein the above-described thermosetting resin is decomposed to provide decomposed products containing a compound, which comprises an acid residue derived from the polyester moiety of the thermosetting resin and a residue derived from the crosslinking moiety of the thermosetting resin (hereinafter, which is referred to as "**compound (I)**"), which is collected, and then recycled. For example, in case that the thermosetting resin is prepared by using of fumaric acid or maleic acid as the polybasic acid, and by using of styrene as the crosslinking agent, the compound (I) of a styrene-fumaric acid copolymer can be collected.
Hereinafter, the method of the preset invention and steps therein are described in order with referring to the attached flowchart (Fig. 1).

Firstly, a thermosetting resin is decomposed by using of subcritical water in the presence of an alkali (Step (A)). Herein, the alkali includes, but is not particularly limited to, (alkali) metals in Group 1A, basic phosphate and the like, which are preferable. Among others, sodium hydroxide and potassium hydroxide are desired from the aspects such as their decomposing ability and their costs. The alkali-concentration of the alkaline aqueous solution is preferably within a range of from 0.5 to 2 N, to which the concentration is not particularly limited.

In this step (A), water is added to the thermosetting resin in the presence of the alkali. Temperature and pressure are increased to allow water to be in a subcritical state in order to decompose the thermosetting resin. Amount of water to be added is preferably within a range of from 200 to 500 parts by weight relative to 100 parts by weight of the thermosetting resin.

During a general plastic decomposition by using of subcritical water, both of the thermal decomposition reaction and the hydrolysis reaction are simultaneously proceeded. It is also applied to the decomposition of the thermosetting plastics made of starting materials including polyalcohol(s) and polybasic acid(s). Herein, the hydrolysis reaction predominantly proceeds. When temperature and/or pressure applied to the subcritical water are appropriately determined, selective hydrolysis reaction is achieved to decompose the thermosetting plastic in order to provide the starting monomers (i.e., polyalcohol(s) and polybasic acid(s) and/or oligomers thereof.

According to the present invention, the thermosetting resin can be treated by contacting with subcritical water to be decomposed to provide polyalcohol(s), polybasic acid(s) and the compound (I). The monomer(s) or oligomer(s) resulted from the decomposition can be collected, and then recycled as materials for newly producing plastics.

The term "subcritical water" herein used means water in a state of that the temperature is no less than 140°C and no more than the critical temperature of water (i.e., 374.4°C) and at a pressure of 0.36 MPa (i.e., saturated vapor pressure at 140°C) or more. Herein, ion product of the subcritical water is about 100 to about 1000 multiple that of water at ambient temperature and ambient pressure. Subcritical water has a decreased dielectric constant equal to those of organic solvents. Subcritical water has an improved wettability to the surface of the thermosetting resin. Subcritical water has these effects accelerating the hydrolysis of the thermosetting resin, which enables to decompose the thermosetting resin to the monomer(s) and/or oligomer(s) thereof.

According to the present invention, temperature of the subcritical water during the decomposition reaction is less than the thermal decomposition temperature of the subjective thermosetting resin to be decomposed, and preferably within a range of from 180 to 300°C. When the temperature during the decomposition reaction is less than 180°C, the cost for the treatment may be increased, since it takes much time to decompose the resin. In this case, yield of the compound (I) tends to be decreased. When the temperature during the decomposition reaction is more than 300°C, the resulting compound (I) can be significantly and thermally decomposed to various slow-molecular derivatives thereof. In this case, it tends to be difficult to collect the compound (I).

Time for the treatment with the subcritical water depends on the treating conditions such as reaction temperature. It is generally within a range of from 1 to 4 hours. Pressure applied to the decomposition reaction system depends on the conditions such as reaction temperature. It is preferably within a range of 2 to 15 MPa.

As it is described above, such decomposition of the thermosetting resin in the subcritical water in the presence of the alkali such as sodium hydroxide and potassium hydroxide can provide an aqueous solution containing a salt of the compound (I), the polyalcohol(s), such as glycol(s), which is/are original monomer(s) in the thermosetting resin, as well as, a salt of the organic acid, such as maleic acid and fumaric acid, which is/are original monomer(s) in the thermosetting resin. The compound (I) has a principle structure comprising the acid residue derived form the polyester moiety of the thermosetting resin and the residue derived from the crosslinking moiety of the thermosetting resin. If the compound (I) is a styrene-fumaric acid copolymer, the acid residue is a fumaric acid residue and the residue derived from the crosslinking moiety is styrene residue. The compound (I) can be formed into a water soluble alkaline salt, wherein the alkali metal (e.g., sodium, potassium) is placed on the carboxyl group of the compound (I) to form a sodium salt (i.e., having the group: -COO⁻Na⁺) or a potassium salt (i.e., having the group: - COO⁻K⁺). The resulting organic acid, such as maleic acid and fumaric acid, may be in a form of alkaline salt, such as sodium salt and potassium salt. Herein, any inorganic materials contained in the thermosetting resin as well as undecomposed thermosetting resin are remaining as solid contents.

The resulting decomposed products are subjected to a solid-liquid separation to provide an aqueous solution containing the alkaline salt of the compound (I) (Step (B)), which is shown in **Fig. 1** attached hereto.

Herein, specifically, the reaction vessel containing the used subcritical water and thus decomposed products is cooled. Subsequently, the contents of the reaction vessel are subjected to a solid-liquid separation by means of a filtration or the like. The separated liquid phase as an aqueous solution contains the alkaline salt of the compound (I), the polyalcohol, and the alkaline salt of the organic acid, each of which is dissolved therein as a water soluble component. The separated solid phase contains the inorganic materials originally contained in the thermosetting resin (e.g., calcium carbonate, glass fiber) and undecomposed thermosetting resin.

An acid is added to the aqueous solution resulted from the step (B) to precipitate the compound (I). Furthermore, a solvent is added to the precipitated compound (1), *with the proviso that* the solvent can dissolve the compound (I) and the solvent is water insoluble. Therefore, the precipitated compound (I) can be dissolved into the solvent, and then collected as a solution containing thereof (Step (C)), which is shown in **Fig. 1** attached hereto.

Herein, specifically, a strong inorganic acid, such as hydrochloric acid, sulfuric acid and nitric acid, is added to the aqueous solution resulted from the step (B) to precipitate the compound (I) as a solid. The precipitated solid compound (I) forms a slurry. The acid is added to the aqueous solution until pH of the aqueous solution is reached to preferably no more than 4 in order to completely precipitate the compound (I) as a solid. It is preferable to add the acid to the aqueous solution until pH of the solution is reached to no more than 2. In the case of that pH value of the solution is further decreased, much more amount of the compound (I) is precipitated as a solid. The lowest pH value is not particularly limited to, but which is more than zero.

Further added to the precipitated compound (I) is a solvent, which can dissolve the compound (I) and which is water insoluble. The precipitated compound (I) is dissolved into the solvent. Preferably, the precipitated compound (I) is dissolved into the solvent, with stirring, under heating at a temperature, for example, within a range of from 70 to 90°C. Subsequently, the solvent phase containing the compound (I) dissolved therein is separated off from the aqueous phase by means of a separatory funnel or the like. The compound (I) can be extracted with the solvent.

Herein, the solvent, which can dissolve the compound (I) and which is water insoluble, includes any solvent which can dissolve the compound (I) and which has a solubility to water (at 25°C) less than 300 g/L, preferably less than 120 g/L.
Such solvent includes, but is not particularly limited to, those satisfy the above-described requirements. Such solvent is selected in consideration of solubility to the compound (I), solubility to water, reactivity to the compound (I), potential of effectively modifying (or esterifying) and recycling the compound (I) to produce a shrinkage inhibitor for a thermosetting resin, which can prevent the shrinking of the uncured thermosetting resin while it is cured, etc. Preferable examples of the solvent include alcohols having 4 or more, preferably 4 to 8 carbon atoms, which may be substituted with a substituent(s) comprising an alkyl group, a cycloalkyl group, a phenyl group and a benzyl group. Among others, primary alcohols and secondary alcohols are preferable.
Saturated alcohols such as 1-butanol, 2-butanol, 1-pentanol, 2-pentanol, 1-hexanol, 2-hexanol, 1-octanol and 2-octanol are preferable from the specific aspects such that these solvents can be used as a modifier for the compound (I) to be recycled, and that the resulting shrinkage inhibitor for a thermosetting resin has an affinity to the other components.
An amount of the solvent to be added is, for example, within a range of from about 1 to about 5 parts by weight, preferably about 2 to about 5 parts by weight, and more preferably about 3 to about 4 parts by weight, relative to the precipitated compound (I) as a basis of weight.
A single solvent can be used alone. Alternatively, two or more solvents can be used in any combination.

In this step (C) of the present invention, a co-solvent may be further added to the aqueous solution resulted from the step (B) together with the solvent which can dissolve the compound (I) and which is water insoluble. The co-solvent can dissolve the compound (I), which is water soluble. The co-solvent can accelerate the dissolution of the compound (I) into the solvent. Therefore, use of such co-solvent can much further effectively collect the compound (I). Herein, the co-solvent, which can dissolve the compound (I) and which is water soluble, includes any solvent which can dissolve the compound (I) and has a solubility to water (at 25°C) no less than 300 g/L, and preferably has any compatibility to water.
Examples of the co-solvent include, but are not particularly limited to, those satisfy the above-described requirements, such as acetone, methyl ethyl ketone, etc. An amount of the co-solvent to be added is, for examples, within a range of from about 1 to about 3 parts by weight relative to the precipitated compound (I) as a basis of weight.
A single co-solvent can be used alone. Alternatively, two or more co-solvents can be used in any combination.

The compound (I) collected by the method according to the present invention can be recycled to produce a shrinkage inhibitor for a thermosetting resin, which can prevent shrinking of the uncured thermosetting resin while it is cured, if the compound (I) is modified to provide the compound (I) with compatibility to the other starting materials for newly producing a thermosetting resin. The alkaline salt of the compound (I) can be recycled and used as a dispersing agent for cements, pigments or the like; a detergent builder; etc.

### Examples

The present invention is further described hereinafter in detail with referring to the following examples. Those skilled in the art will appreciate the present invention is not limited to those examples.

### Example 1

Glycols of propylene glycol, neopentyl glycol and dipropylene glycol, and maleic anhydride in a stoichiometric amount to the glycols were subjected to a polycondensation to synthesize an unsaturated polyester. Styrene as a crosslinking agent was added to the neat varnish of the unsaturated polyester in a stoichiometric amount relative to the polyester to give a liquid resin. 165 Parts by weight of calcium carbonate and 90 parts by weight of glass fiber were added to 100 parts by weight of the liquid resin. The liquid resin was cured in a mold to give a molded unsaturated polyester resin product (hereinafter, which is referred to as "**thermosetting resin**").

4 g of the thermosetting resin and 16 g of a 1N NaOH aqueous solution were charged into a reaction tube. The reaction tube was immersed into a bath at a constant temperature of 230°C. The reaction tube immersed in the bath was left for 2 hours, wherein the water was in the subcritical state. The thermosetting resin was decomposed.

Subsequently, the reaction tube was taken up from the bath, and then immersed into a cooling bath. The reaction tube was immediately cooled, and then heated to the room temperature. After the decomposition treatment, the reaction tube contained components dissolved in water, undissolved resin residue, calcium carbonate and glass fiber. The contents in the reaction tube were filtrated to separate off the solid contents to give an aqueous solution.

Subsequently, a 1N sulfuric acid was added to the aqueous solution, until pH of the solution was reached to 2, to precipitate the styrene-fumaric acid copolymers. Added to the precipitated styrene-fumaric acid copolymers were 1.86 parts by weight of 1-octanol (having a boiling point of 195°C), as a solvent, relative to 1.00 part by weight of the styrene-fumaric acid copolymers. The styrene-fumaric acid copolymers were extracted with 1-octanol, with stirring, under heating at 90°C.

Subsequently, the mixture was left for 1 hour to separate the upper solvent (i.e., 1-octanol) phase and the bottom aqueous phase. The bottom aqueous phase was removed off to give a remaining 1-octanol solution containing the styrene-fumaric acid copolymers.

The extraction rate of the styrene-fumaric acid copolymers into the solvent phase was calculated according to the following equation.

[Extraction rate of the styrene-fumaric acid copolymers into the solvent phase] = [(weight of the styrene-fumaric acid copolymers contained in the aqueous solution) - (weight of the styrene-fumaric acid copolymers attached to the vessel used for the extraction) - (weight of the styrene-fumaric acid copolymers contained in the aqueous phase)] / [weight of the styrene-fumaric acid copolymers contained in the aqueous solution]

Herein, added to the above-described separated liquid phase was the acid to precipitate the styrene-fumaric acid copolymers contained therein. The precipitated styrene-fumaric acid copolymers were subjected to a solid-liquid separation to be separated off. The separated styrene-fumaric acid copolymers were dried, and then weighted. The determined weight of the styrene-fumaric acid copolymers is herein referred to as the "weight of the styrene-fumaric acid copolymers contained in the aqueous solution" in the above-represented equation.
The styrene-fumaric acid copolymers attached to the vessel used for the extraction were washed off with acetone or methanol. The acetone or methanol containing the styrene-fumaric acid copolymers was collected, and then evaporated off to give the styrene-fumaric acid copolymers. The remaining styrene-fumaric acid copolymers were dried, and then weighted. The determined weight of the styrene-fumaric acid copolymers is herein referred to as the "weight of the styrene-fumaric acid copolymers attached to the vessel used for the extraction" in the above-represented equation.
The aqueous phase separated off from the solvent phase was filtered to collect the styrene-fumaric acid copolymers, which was contained in the aqueous phase. The collected styrene-fumaric acid copolymers were dried, and then weighted. The determined weight of the styrene-fumaric acid copolymers is herein referred to as the "weight of the styrene-fumaric acid copolymers contained in the aqueous phase" in the above-represented equation.

### Example 2

The procedures according to the Example 1 were carried out under the conditions of the Example 1, except that the solvent was 1-octanol (having a boiling point of 195°C), wherein 4 parts by weight of 1-octanol were added to the precipitated styrene-fumaric acid copolymers as a basis of the weight. The styrene-fumaric acid copolymers were extracted with 1-octanol, with stirring, under heating at 90°C. The extraction rate of the styrene-fumaric acid copolymers into 1-octanol phase was determined.

### Example 3

The procedures according to the Example 1 were carried out under the conditions of the Example 1, except that the solvent was 1-butanol (having a boiling point of 117°C), wherein 1.86 parts by weight of 1-butanol were added to 1.00 part by weight of the precipitated styrene-fumaric acid copolymers. The styrene-fumaric acid copolymers were extracted with 1-butanol, with stirring, under heating at 90°C. The extraction rate of the styrene-fumaric acid copolymers into 1-butanol phase was determined.

### Example 4

The procedures according to the Example 1 were carried out under the conditions of the Example 1, except that acetone, as a co-solvent, was used in addition to the solvent of 1-octanol, wherein 1.5 parts by weight of acetone were added together with the solvent of 1-octanol relative to 1.00 part by weight of the precipitated styrene-fumaric acid copolymers. The extraction rate of the styrene-fumaric acid copolymers into 1-octanol phase was determined.

Table 1 shows conditions in the examples and results such as extraction rate of the styrene-fumaric acid copolymers.

**Table 1**

| | **Solvent** | **Amount of solvent¹⁾** | **Co-solvent** | **Amount of co-solvent²⁾** | **Extraction rate³⁾** |
|---|---|---|---|---|---|
| **Example 1** | 1-octanol | 1.86 | none | - | 0.72 |
| **Example 2** | 1-octanol | 4.00 | none | - | 0.95 |
| **Example 3** | 1-butanol | 1.86 | none | - | 0.68 |
| **Example 4** | 1-octanol | 1.86 | acetone | 1.50 | 0.96 |

| | | | | | |
|---|---|---|---|---|---|
| 1) Amount of the solvent added to the styrene-fumaric acid copolymers as a basis of weight (in parts by weight) relative to 1.00 part by weight of the styrene-fumaric acid copolymers 2) Amount of the co-solvent added to the styrene-fumaric acid copolymers as a basis of weight (in parts by weight) 3) Extraction rate of the styrene-fumaric acid copolymers into the solvent phase | | | | | |

As the results are shown in the Table 1, the Examples 1-4 demonstrate that the solvent which can dissolve the styrene-fumaric acid copolymers and which is water insoluble was added to the styrene-fumaric acid copolymers to allow the styrene-fumaric acid copolymers to be extracted with the solvent, and therefore the styrene-fumaric acid copolymers can be effectively collected. Among others, the Example 4 by using of the co-solvent demonstrates that the styrene-fumaric acid copolymers can be collected with a much higher extraction rate.
The Example 2, wherein 4.00 parts by weight of the solvent is added to the precipitated styrene-fumaric acid copolymers as a basis of weight, demonstrates that the styrene-fumaric acid copolymers can be collected with a higher extraction rate.

## Claims

1. A method for decomposing a thermosetting resin comprising a polyester moiety and a crosslinking moiety therewith to provide decomposed products to be collected and recycled, which comprises steps of:
(A) decomposing the thermosetting resin by using of subcritical water in the presence of an alkali
(B) subjecting the decomposed products to a solid-liquid separation to provide an aqueous solution comprising a salt of a compound with an alkali, wherein the compound comprises an acid residue derived from the polyester moiety and a residue derived from the crosslinking moiety; and
(C) adding an acid to the aqueous solution to precipitate the compound, and adding thereto a solvent, which can dissolve the compound and which is water insoluble, to dissolve the precipitated compound into the solvent to provide a solution comprising the compound to be collected.

2. The method according to claim 1, wherein the solvent, which can dissolve the compound and which is water insoluble, comprises an alcohol.

3. The method according to claim 1 or 2, wherein the step (c) further comprises adding a co-solvent, which can dissolve the compound and which is water soluble, together with the solvent, which can dissolve the compound and which is water insoluble.
